# FASCICULE DE BREVET EUROPEEN

(11) **EP 2 646 794 B1**
(45) Date de publication et mention de la délivrance du brevet: **27.08.2014**
(21) Numéro de dépôt: 11788185.4
(22) Date de dépôt: 28.11.2011
(51) Int. Cl.: G01N 1/22, G01N 1/40, G01N 33/22

(54) **PROCÉDÉ POUR DÉTECTER IN SITU LA PRÉSENCE D'EXPLOSIFS DANS UN MILIEU GAZEUX**
VERFAHREN ZUR IN SITU DETEKTION VON SPRENGSTOFFEN IN EINEM GASFÖRMIGEN MEDIUM
METHOD FOR IN SITU DETECTION OF EXPLOXSIVES IN A GASEOUS MEDIUM

(30) Priorité: 29.11.2010 FR 1059852
(43) Date de publication de la demande: 09.10.2013
(73) Titulaire: Commissariat à l'Énergie Atomique et aux Énergies Alternatives, 75015 Paris (FR)
(72) Inventeur: BRY, Alain, 37190 VALLERES (FR); BOUSQUET, Marilyne, F-37510 Savonnieres (FR); HAIRAULT, Lionel, F-37150 Blere (FR); MAILLOU, Thierry, F-37300 Joue Les Tours (FR)
(74) Mandataire: Ilgart, Jean-Christophe
(86) Numéro de dépôt international: PCT/EP2011/071188
(87) Numéro de publication internationale: WO 2012/072582

(56) Documents cités:
- WO-A-2010/044920
- FR-A1- 2 859 393
- US-A- 3 960 523
- US-A- 4 595 399
- US-A- 5 011 517
- US-A- 5 914 454
- WESLEY R. COFER ET AL: "Improved aqueous scrubber for collection of soluble atmospheric trace gases", ENVIRONMENTAL SCIENCE & TECHNOLOGY, vol. 19, no. 6, 1 juin 1985 (1985-06-01), pages 557-560, XP055001744, ISSN: 0013-936X, DOI: 10.1021/es00136a012

## Description

### DOMAINE TECHNIQUE

L'invention se rapporte à un procédé permettant de détecter *in situ,* avec une très grande sensibilité et de manière immédiate ou quasi immédiate, la présence d'au moins un explosif dans un milieu gazeux et ce, que cet explosif soit présent à l'état gazeux, liquide et/ou solide dans ce milieu gazeux.

Ce procédé est notamment susceptible d'être utilisé pour assurer la sécurité civile, en particulier dans des lieux publics considérés comme sensibles comme les aéroports, les gares et les stations de métro, pour détecter des activités clandestines de fabrication d'explosifs ou pour surveiller à des fins sécuritaires des sites industriels fabriquant, entreposant et/ou manipulant des explosifs.

### ÉTAT DE LA TECHNIQUE ANTÉRIEURE

La détection d'explosifs est un problème d'intérêt crucial, notamment en matière de sécurité civile.

Le cas particulier de la détection d'explosifs présents dans l'atmosphère est extrêmement délicat en raison de la faible tension de vapeur de ces composés. Ainsi, par exemple, le 2,4,6-trinitrotoluène (TNT) a une tension de vapeur de quelques ppb (partie par milliard, soit une molécule parmi un milliard de molécules d'air), tandis que la cyclotriméthylène-trinitramine, encore appelée hexogène ou RDX, a une tension de vapeur de quelques ppt (partie par trillion, soit une molécule parmi mille milliards de molécules d'air).

Ce qui signifie que le système de détection doit, non seulement être mobile de sorte à pouvoir être utilisé sur site si l'on veut obtenir une identification rapide, voire immédiate ou quasi immédiate des explosifs présents dans l'atmosphère, mais qu'il doit aussi être extrêmement sensible.

L'une des méthodes utilisées pour détecter des explosifs présents dans l'atmosphère, qui présente l'avantage d'être à la fois mobile et d'une grande sensibilité, consiste à employer des chiens dits « renifleurs », dressés et entraînés à cet effet. Toutefois, cette méthode présente aussi l'inconvénient de nécessiter une formation longue et continue des chiens et de leurs maîtres, et d'être inadaptée à des opérations prolongées en raison de ce que la durée d'attention des chiens est relativement limitée.

On connaît, par ailleurs, un certain nombre d'appareils mobiles, voire portables, conçus pour détecter la présence d'explosifs dans l'atmosphère, parmi lesquels on peut citer :
- les détecteurs développés par la société Smiths Detection d'une part, et par la société GE Ion Track, Inc. d'autre part, et dont le fonctionnement est basé sur la spectrométrie à mobilité ionique ;
- les détecteurs développés par la société Scintrex Trace Corp. et dont le fonctionnement est basé sur la réduction des oxydes d'azote NO₂, qui sont issus de la décomposition des explosifs, en NO et sur la mesure du courant électrique produit par cette réduction ; et
- les détecteurs développés par l'équipe de R. Graham Cooks à l'Université de Purdue (Indiana, USA) qui utilisent la désorption-ionisation par électrospray (DESI).

Dans la pratique, le coût élevé de ces appareils (de l'ordre de 20 000 euros/pièce) et leur faible niveau de fiabilité restent un frein à une généralisation de leur usage.

On connaît également, par le brevet US numéro 5,011,517 **[1],** un procédé pour détecter la présence de vapeurs, notamment d'explosifs, dans un gaz par extraction et concentration de ces vapeurs dans un milieu liquide, qui consiste à mettre en contact, sous la forme d'un tourbillon, ce gaz avec un liquide de nettoyage apte à capter ces vapeurs.

On connaît aussi, par Cofer III et al., Envir. Sci. Technol. 1985, 19(6), 557-560 **[2],** un procédé pour collecter les gaz qui sont présents à l'état de traces dans l'air atmosphérique, par extraction et concentration de ces gaz dans un milieu liquide, qui consiste à nébuliser le milieu liquide au moyen de cet air et à condenser les gouttelettes du brouillard résultant par contact avec une membrane hydrophobe.

Pour résoudre un tout autre problème que celui de la détection de la présence d'explosifs dans l'atmosphère, il a été proposé, dans la demande internationale PCT publiée sous le numéro WO 2005/025721 **[3],** un procédé qui permet d'extraire très efficacement des polluants gazeux, liquides et/ou solides d'un milieu gazeux et de les concentrer dans un milieu liquide, par nébulisation de ce milieu liquide au moyen du milieu gazeux et condensation des gouttelettes formant le brouillard produit par cette nébulisation, et qui peut être mis en oeuvre dans un appareil compact, se présentant sous la forme d'un coffret et donc utilisable sur site.

Or, dans le cadre de leurs travaux, les inventeurs ont constaté que l'utilisation de ce procédé, en combinaison avec des réactifs convenablement choisis, peut être mise à profit pour détecter, avec une très grande sensibilité et de manière immédiate ou quasi immédiate, la présence d'explosifs dans l'atmosphère et, qui plus est, de manière continue.

Et c'est sur cette constatation qu'est basée la présente invention.

### EXPOSÉ DE L'INVENTION

L'invention a donc pour objet un procédé pour détecter *in situ* la présence d'au moins un explosif dans un milieu gazeux, qui comprend une opération d'extraction et de concentration de cet explosif dans un milieu liquide, cette opération comprenant la nébulisation du milieu liquide au moyen du milieu gazeux et la condensation des gouttelettes de milieu liquide formant le brouillard produit par cette nébulisation, et qui est caractérisé en ce que :
- on utilise, comme milieu liquide, un milieu qui contient au moins une substance capable de réagir avec cet explosif ou avec un produit résultant de la dégradation de cet explosif par un agent chimique, et dont la réaction ou l'interaction avec ledit explosif ou ledit produit de dégradation entraîne une variation d'au moins un paramètre physico-chimique du milieu liquide ;
- on mesure ledit paramètre physico-chimique pendant l'opération d'extraction et de concentration de l'explosif ou à la fin de cette opération ; et
- on corrèle le résultat de la mesure dudit paramètre physico-chimique avec la présence ou l'absence de l'explosif dans le milieu gazeux.

Dans le cadre de la présente invention, le terme « explosif » englobe les explosifs intrinsèques, c'est-à-dire les composés qui sont porteurs de groupements chimiques explosophores du type NO₂, ONO₂, NO, N-NO₂, N=N, N=N=N, N-halogène, N-soufre, N=C, OClO₂, OClO₃, O-O, O-O-O, C≡C, C-métal, et qui sont donc capables de se décomposer de manière explosive sous l'effet d'une impulsion telle qu'un impact ou une variation de température.

Il englobe également les précurseurs de ces explosifs, c'est-à-dire les composés qui sont utilisés dans les réactions chimiques par lesquelles ils sont produits et les constituants de compositions explosives. C'est le cas par exemple du nitrométhane.

Il englobe encore les marqueurs d'explosifs au sens de la « Convention sur le Marquage des Explosifs Plastiques et en Feuilles aux Fins de Détection » signée à Montréal le 1er mars 1991. On rappelle que par « marqueur d'explosif », cette Convention entend une substance qui est ajoutée à un explosif pour le rendre détectable. Sont cités comme marqueurs d'explosifs dans cette Convention, le dinitrate d'éthylèneglycol (EGDN), le 2,3-diméthyl-2,3-dinitrobutane (DMNB) et les isomères para et ortho du nitrotoluène (*p*-MNT et o-MNT).

Ainsi, à titre d'exemples non limitatifs d'explosifs susceptibles d'être détectés par le procédé de l'invention, on peut citer :
- les explosifs relevant de la famille des composés nitroaromatiques tels que le trinitrophénol (ou acide picrique), le TNT, le 2,4-dinitrotoluène (2,4-DNT), les isomères du nitrotoluène précédemment évoqués, le 2-amino-4,6-dinitrotoluène (ADNT), le 1,3,5-trinitrobenzène (TNB), le 1,3-dinitrobenzène (1,3-DNB), le nitrobenzène, le 2,4-dinitrofluorobenzène (DNFB), le 1-chloro-2,6-dinitrotrifluorométhylbenzène, le 2,4-dinitrotrifluorométhoxybenzène (DNTFMB) et l'hexanitrostilbène (HNS) ;
- les explosifs relevant de la famille des nitramines ou nitrosamines tels que l'hexogène précédemment évoqué, la cyclotétraméthylène-tétranitramine (plus simplement appelée octogène ou HMX), la 2,4,6-trinitrophénylméthylnitramine (plus simplement appelée tétryle) et l'hexamine (qui est un précurseur de l'hexogène) ;
- les explosifs relevant de la famille des esters nitriques tels que le tétranitrate de penta-érythritol (plus simplement appelé pentrite), le trinitrate de glycérol (plus simplement appelé nitroglycérine), la nitroguanidine, le nitrate de cellulose (plus simplement appelé nitrocellulose) et l'EGDN précédemment évoqué ; et
- les explosifs relevant de la famille des peroxydes organiques tels que le triperoxyde de triacétone, l'hexaméthyltriperoxyde et le peroxyde d'hydrogène (qui est un précurseur du triperoxyde de triacétone).

Par ailleurs, dans le cadre de la présente invention, l'expression « détecter la présence » couvre aussi bien le fait d'identifier et de quantifier un explosif que celui d'identifier cet explosif sans le quantifier.

Conformément à l'invention, le paramètre physico-chimique du milieu liquide, dont la variation est induite par la réaction ou l'interaction entre la substance et l'explosif ou le produit résultant de sa dégradation par l'agent chimique, peut être une propriété optique comme la densité optique, la fluorescence ou la luminescence, ou une propriété électrique, en l'espèce la conductivité, ou encore une propriété électrochimique comme le degré d'oxydation ou de réduction.

Toutefois, on préfère que ce paramètre physico-chimique soit une propriété optique et, plus encore, que cette propriété optique soit la densité optique (ou absorbance), qui est aisément mesurable par spectrophotométrie, ou la fluorescence, qui, elle, est aisément mesurable par fluorimétrie.

De ce fait, la substance contenue dans le milieu liquide est préférentiellement choisie parmi les substances dont la réaction ou l'interaction avec l'explosif ou le produit résultant de sa dégradation par l'agent chimique conduit à la formation d'un produit de réaction ou d'un complexe dont le spectre d'absorption UV-visible ou de fluorescence est différent de celui que présente la substance elle-même et dont la présence dans le milieu liquide se traduit, par conséquent, par une variation de la densité optique ou de la fluorescence de ce milieu.

Ainsi, la substance contenue dans le milieu liquide peut notamment être choisie parmi les bases fortes telles que l'hydroxyde de sodium, l'hydroxyde de potassium, l'hydroxyde de calcium ou l'hydroxyde de tributylammonium, et les halogénures de tributylammonium du type fluorure, iodure ou bromure, qui réagissent avec un solvant protique tel que l'acétone ou l'acétonitrile pour former, avec un composé nitro-aromatique, l'anion Janowsky correspondant.

La substance contenue dans le milieu liquide peut également être choisie parmi les composés de titane(IV) tels que, par exemple, l'oxosulfate de titane(IV), le tétrachlorure de titane(IV), les orthotitanates de tétraalkyle comme l'orthotitanate de tétraméthyle, l'orthotitanate de tétraéthyle, l'orthotitanate de tétra-n-propyle ou l'orthotitanate de tétra-*i*-propyle, qui forment avec le peroxyde d'hydrogène des complexes de couleur jaune aisément détectables par spectrophotométrie.

Parmi ces composés, l'oxosulfate de titane(IV) est particulièrement préféré.

Les composés de titane(IV) peuvent être utilisés en premier lieu pour détecter le peroxyde d'hydrogène.

Toutefois, ils peuvent également être utilisés pour détecter tout explosif de la famille des peroxydes qui est susceptible d'être dégradé par un agent chimique en libérant du peroxyde d'hydrogène.

Ceci est notamment le cas du triperoxyde de triacétone qui libère du peroxyde d'hydrogène lorsqu'il est attaqué par un acide fort tel que l'acide sulfurique, nitrique, chlorhydrique ou *para-*toluènesulfonique.

Pour détecter de tels peroxydes, on utilise donc comme milieu liquide, un milieu qui contient, non seulement au moins un composé de titane(IV), mais également au moins un agent chimique propre à dégrader ces peroxydes en peroxyde d'hydrogène, par exemple de l'acide sulfurique si le peroxyde à détecter est le triperoxyde de triacétone.

La substance contenue dans le milieu liquide peut encore être choisie parmi les phtalocyanines, qui sont des molécules fluorescentes et dont la fluorescence s'éteint lorsqu'elles sont en présence de composés nitroaromatiques tels que le TNT et le 2,4-DNT.

Parmi les phtalocyanines, on préfère tout particulièrement celles qui répondent à la formule (I) ci-après : 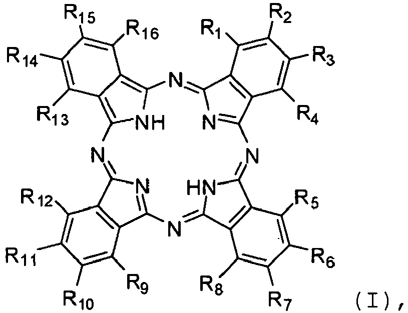 dans laquelle :
- R₁, R₄, R₅, R₈, R₉, R₁₂, R₁₃ et R₁₆, identiques ou différents, représentent un atome d'hydrogène ou un groupe alkyle comprenant de 1 à 4 atomes de carbone et, mieux encore, un groupe méthyle ou éthyle, tandis que R₂, R₃, R₆, R₇, R₁₀, R₁₁, R₁₄ et R₁₅ représentent un groupe alcoxy, notamment octoxy (O(CH₂)₇CH₃) ; ou bien
- R₁, R₂, R₄, R₅, R₆, R₈, R₉, R₁₀, R₁₂, R₁₃, R₁₄ et R₁₆ représentent un atome d'hydrogène, tandis que R₃, R₇, R₁₁ et R₁₄ représentent un groupe aryloxy, notamment phénoxy, ou un groupe silylé, notamment diméthyl-hydroxysilyle ;
ces phtalocyanines pouvant être métallées, notamment par du zinc, ou non métallées.

La substance contenue dans le milieu liquide peut en outre être choisie parmi les coumarines telles que, par exemple, la coumarine *stricto sensu* (ou 2*H*-1-benzopyran-2-one), l'ombelliférone (ou 7-hydroxy-coumarine), l'esculétol (ou 6,7-dihydroxycoumarine), le scopolétol (ou 6-méthoxy-7-hydroxycoumarine) ou le fraxétol (ou 6-méthoxy-7,8-dihydroxycoumarine), et le dansylamide, dont la fluorescence s'éteint lorsqu'elles sont en présence de composés nitroaromatiques tels que le TNT et le 2,4-DNT.

Comme précédemment évoqué, le milieu liquide peut contenir au moins un agent chimique capable de dégrader l'explosif que l'on cherche à détecter.

Il peut en outre contenir au moins un composé propre à favoriser la solubilisation dans le milieu liquide de l'explosif ou du produit résultant de sa dégradation par l'agent chimique. Typiquement, ce composé est représenté par le solvant ou le mélange de solvants dans lequel est dissoute la substance destinée à réagir ou à interagir avec l'explosif ou le produit résultant de sa dégradation par l'agent chimique et qui est choisi en fonction de la solubilité que présente ledit explosif ou ledit produit de dégradation dans l'eau et les solvants organiques.

Eu égard à ce qui précède, la mesure du paramètre physico-chimique du milieu liquide est, de préférence, réalisée par spectrophotométrie ou par fluorimétrie.

Conformément à l'invention, il est possible d'attendre la fin de l'opération d'extraction et de concentration de l'explosif dans le milieu liquide pour effectuer cette mesure. Il est également possible d'effectuer cette mesure à intervalles de temps, réguliers ou non, pendant que se déroule l'opération d'extraction.

Toutefois, dans le cadre de l'invention, on préfère effectuer cette mesure en continu pendant toute la durée de l'opération d'extraction et de concentration de l'explosif dans le milieu liquide de manière à pouvoir être alerté sur la présence de l'explosif dans le milieu gazeux dès qu'il se produit une variation de la propriété physico-chimique du milieu liquide significative de cette présence.

Quand à la corrélation entre le résultat de la mesure dudit paramètre physico-chimique avec la présence ou l'absence de l'explosif dans le milieu gazeux, elle est réalisée en comparant ce résultat à un ou plusieurs blancs, c'est-à-dire aux résultats de mesures ayant été préalablement réalisées sur un milieu gazeux de même nature (par exemple, de l'air si le milieu gazeux dont on cherche à savoir s'il renferme des explosifs est l'air ambiant) mais exempt de toute trace d'explosif.

Conformément à l'invention, l'opération d'extraction et de concentration de l'explosif dans le milieu liquide comprend une première et une deuxième condensation des gouttelettes de milieu liquide formant le brouillard produit par la nébulisation, la première condensation étant réalisée par coalescence des gouttelettes sur une surface mouillée et la deuxième condensation étant réalisée par contact desdites gouttelettes avec une surface froide.

Pour ce faire, le procédé de l'invention est, de préférence, mis en oeuvre dans un appareil qui comprend :
- une première enceinte de nébulisation et de condensation, qui présente une partie supérieure et une partie inférieure destinée à contenir le milieu liquide, et qui est munie d'un conduit d'amenée du milieu gazeux, de moyens pour nébuliser le milieu liquide, et d'un premier conduit d'évacuation du milieu gazeux ;
- des moyens de mise en dépression ou en surpression de l'intérieur de la première enceinte pour permettre au milieu gazeux de pénétrer dans cette première enceinte, d'y circuler et d'en être évacué, en un flux continu ;
- une deuxième enceinte de condensation, cette deuxième enceinte étant reliée au conduit d'évacuation du milieu gazeux de la première enceinte, et étant munie d'un deuxième conduit d'évacuation du milieu gazeux ;
- des moyens pour refroidir cette deuxième enceinte ; et
- des moyens pour mesurer le paramètre physico-chimique du milieu liquide dont la variation est induite par la réaction ou l'interaction entre la substance et l'explosif ou le produit résultant de sa dégradation par l'agent chimique.

De préférence, ces moyens de mesure sont des moyens de mesure spectrophotométrique et/ou fluorimétrique.

Le procédé de l'invention présente de nombreux avantages. En effet, outre de permettre la détection instantanée ou quasi instantanée d'explosifs présents dans un milieu gazeux même lorsque ceux-ci sont présents en quantité extrêmement faible - comme cela sera démontré ci-après -, il est susceptible d'être mis en oeuvre dans un appareil qui, si on le souhaite, peut se présenter sous une forme compacte, le rendant transportable et utilisable sur tout type de site, et dont le coût de fabrication est bien moins élevé que celui des détecteurs proposés à ce jour dans l'état de la technique.

D'autres avantages et caractéristiques de l'invention apparaîtront à la lecture du complément de description qui suit, qui se rapporte à un exemple d'appareil susceptible d'être utilisé pour la mise en oeuvre du procédé de l'invention ainsi qu'à des tests de détection d'explosifs ayant été réalisés avec cet appareil et ayant permis de valider le procédé de l'invention.

Il va de soi, toutefois, que ce complément de description n'est donné qu'à titre d'illustration de l'objet selon l'invention et ne constitue en aucune manière une limitation de cet objet.

### BRÈVE DESCRIPTION DES DESSINS

La Figure 1 est un schéma de principe d'un exemple d'appareil susceptible d'être utilisé pour la mise en oeuvre du procédé de l'invention, vu en coupe transversale.
La Figure 2 représente schématiquement une partie de l'appareil montré sur la Figure 1.
La Figure 3 montre les résultats d'un premier test de détection d'un explosif par le procédé de l'invention, ayant été réalisé avec des vapeurs de 2,4-dinitrotoluène diluées dans l'air ambiant.
La Figure 4 montre les résultats d'un deuxième test de détection d'un explosif par le procédé de l'invention, ayant été réalisé avec des vapeurs de peroxyde d'hydrogène diluées dans l'air ambiant.
La Figure 5 montre les résultats d'un troisième test de détection d'un explosif par le procédé de l'invention, ayant été réalisé avec des vapeurs de triperoxyde de triacétone diluées dans l'air ambiant.

### EXPOSÉ DÉTAILLÉ DE MODES DE RÉALISATION PARTICULIERS

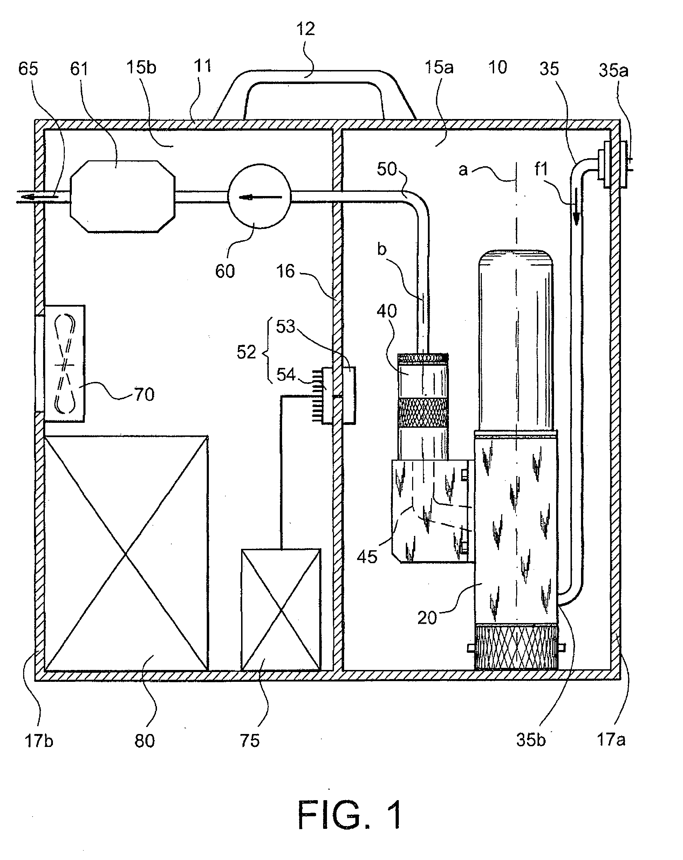

On se réfère tout d'abord à la Figure 1 qui correspond à un schéma de principe d'un exemple d'appareil susceptible d'être utilisé dans le procédé de l'invention.

Dans cet exemple, l'appareil, qui a la référence 10, se présente sous une forme compacte, précisément sous la forme d'un coffret 11 prévu pour être portée par une seule personne grâce à une poignée 12 disposée sur sa face externe supérieure.

Comme visible sur la Figure 1, l'intérieur de la mallette 11 est divisé en deux compartiments, respectivement 15a et 15b, par une cloison verticale 16.

Le compartiment 15a renferme :
- une première enceinte 20, de grand axe a vertical, qui est destinée à assurer à la fois la nébulisation d'un milieu liquide par le milieu gazeux dont on cherche à déterminer s'il contient un ou des explosifs, ainsi que la condensation par coalescence d'une partie du brouillard formé par cette nébulisation ;
- un conduit 35, qui sert à amener le milieu gazeux dans la première enceinte 20 et qui, pour ce faire, a une extrémité 35a qui est située hors de la mallette 11 et une extrémité 35b qui est située dans la première enceinte 20 ;
- une deuxième enceinte 40, également de grand axe b vertical, dont la fonction est d'assurer la condensation thermique du brouillard ne s'étant pas condensé dans la première enceinte 20 ;
- un conduit 45, qui relie les enceintes 20 et 40 l'une à l'autre, et qui sert notamment à évacuer le milieu gazeux hors de la première enceinte 20 ; et
- un conduit 50, qui sert à évacuer le milieu gazeux hors de la deuxième enceinte 40 et qui, pour ce faire, relie cette enceinte à une pompe aspirante 60 située dans le compartiment 15b, en traversant la cloison 16.

Le compartiment 15a renferme également un dispositif dont la fonction est de refroidir la deuxième enceinte 40 pour permettre la condensation thermique du brouillard ne s'étant pas condensé dans la première enceinte 20 et, éventuellement, la première enceinte 20 pour limiter une volatilisation du milieu liquide si celui-ci est à base d'un ou de solvants volatiles et éviter ainsi une perte de solvant(s).

Dans le mode de réalisation illustré sur la Figure 1, ce dispositif est une cellule à effet Peltier 52 dont la face, qui est destinée à servir de face froide 53 en condition de fonctionnement de l'appareil, se situe au contact de la cloison 16 et est dirigée vers l'intérieur du compartiment 15a, tandis que la face, qui est destinée à servir de face chaude 54, se situe dans le compartiment 15b.

Le compartiment 15b renferme, lui :
- la pompe aspirante 60, qui est, de préférence, une pompe volumétrique et dont la fonction est de mettre en dépression l'intérieur des enceintes 20 et 40 et de permettre, ainsi, au milieu gazeux de pénétrer dans ces enceintes, d'y circuler et d'en être évacué en un flux continu ;
- un conduit 65, dont la fonction est d'évacuer le milieu gazeux hors de la mallette 11 et qui, pour ce faire, relie la pompe aspirante 60 à l'extérieur de la mallette 11 en traversant la paroi latérale 17b de cette dernière ;
- un débitmètre 61 couplé à la pompe aspirante 60, qui sert à mesurer le débit du flux gazeux traversant les enceintes 20 et 40 ;
- un bloc de ventilation 70, qui sert à évacuer hors du compartiment 15b la chaleur dégagée par la face chaude de la cellule à effet Peltier 52 dans ce compartiment ;
- des circuits électroniques programmés (non représentés sur la Figure 1), qui sont gérés par une unité de contrôle et de commande 75 pour le pilotage et la régulation du fonctionnement de l'appareil 10 ; et
- un système d'alimentation en énergie électrique 80, qui est notamment destiné à alimenter la cellule à effet Peltier 52, la pompe aspirante 60, le débitmètre 61, le compteur 62, le bloc de ventilation 70, les circuits électroniques programmés et l'unité centrale de contrôle et de commande 75, ce système d'alimentation comprenant avantageusement des moyens permettant de raccorder l'appareil à un réseau électrique ou à des batteries externes tel qu'un câble de connexion, et, d'autre part, des moyens du type batteries, accumulateurs rechargeables ou analogues, le rendant apte à fonctionner de façon autonome.

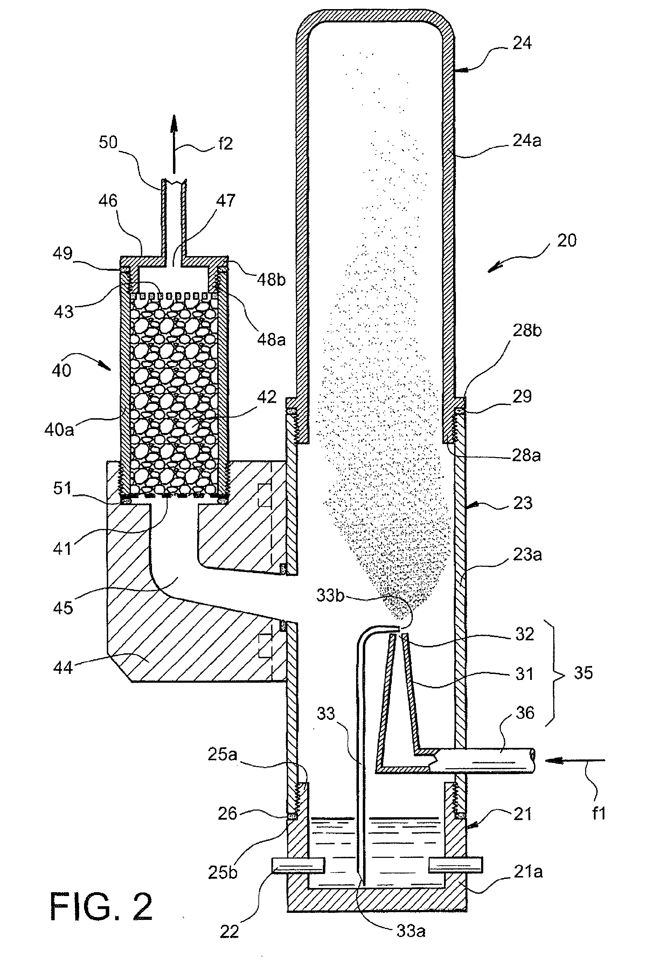

On se réfère à présent à la Figure 2 qui représente, sous une forme schématique, les éléments de l'appareil 10 présents dans le compartiment 15a, à l'exception toutefois de la face froide 53 de la cellule à effet Peltier 52.

On retrouve donc, sur cette figure, la première enceinte 20, le conduit 35 servant à amener le milieu gazeux dans cette première enceinte, la deuxième enceinte 40, le conduit 45 reliant les deux enceintes 20 et 40, ainsi que le conduit 50 servant à évacuer le milieu gazeux hors de la deuxième enceinte.

Comme visible sur la Figure 2, la première enceinte 20 se compose de trois parties qui sont, de bas en haut :
- un réservoir 21, qui forme la partie inférieure de cette enceinte et qui est rempli, avant chaque opération d'extraction-concentration, d'un volume prédéterminé du milieu liquide choisi pour réaliser cette opération ;
- une chambre de nébulisation 23, qui forme la partie intermédiaire de cette enceinte ; et
- un dôme de condensation 24, qui forme la partie supérieure de cette enceinte.

Le réservoir 21, la chambre de nébulisation 23 et le dôme de condensation 24 présentent chacun une paroi, respectivement 21a, 23a et 24a, délimitant un cylindre à section droite circulaire, la paroi 23a du réservoir 21 étant fermée à son extrémité inférieure, tandis que la paroi 24a du dôme de condensation est fermé à son extrémité supérieure.

Ces parois sont munies de filetages de forme et de dimensions conjuguées qui permettent d'assembler et de désassembler à volonté le réservoir 21, la chambre de nébulisation 23 et le dôme de condensation par simple vissage.

La première enceinte 20 renferme des moyens de nébulisation. Ces moyens comprennent :
- d'une part, une buse de nébulisation 31, qui est formée par une réduction progressive de la section de la partie du conduit 35 se trouvant à l'intérieur de la chambre de nébulisation 23 de manière à augmenter, en condition de fonctionnement de l'appareil, la vitesse à laquelle le milieu gazeux est libéré dans cette chambre par rapport à la vitesse d'écoulement du milieu gazeux dans le conduit 35, et qui est terminée par un orifice 32 ; et
- d'autre part, un tuyau de nébulisation 33, qui est disposé verticalement dans la première enceinte tout en étant courbé à sa partie supérieure, de sorte que son ouverture inférieure 33a puisse plonger dans le milieu liquide contenu dans le réservoir 21 en condition de fonctionnement de l'appareil, tandis que son ouverture supérieure 33b, qui présente une section sensiblement inférieure à celle présentée par le reste de ce tuyau, se situe juste au-dessus de l'orifice 32 de la buse de nébulisation.

Comme visible aussi sur la Figure 2, la deuxième enceinte 40 présente une paroi 40a délimitant un cylindre à section droite circulaire et qui est ouverte à ses deux extrémités.

Au niveau de son extrémité inférieure, la paroi 40a est munie d'une grille qui forme la base de la deuxième enceinte 40 et dont les ouvertures permettent de laisser passer, en condition de fonctionnement de l'appareil, le brouillard provenant de la première enceinte 20 ainsi que les gouttes qui se forment dans la deuxième enceinte 40 de par la condensation thermique de ce brouillard, tout en retenant un garnissage 42 du type cylindres en polyétheréthercétone, billes de polytétrafluoroéthylène (Teflon^{®}), billes en acier inoxydable ou anneaux de Raschig, qui est logé dans cette deuxième enceinte.

Ce garnissage, dont la fonction est d'augmenter la surface froide sur laquelle le brouillard est susceptible de se condenser en condition de fonctionnement de l'appareil et d'optimiser ainsi la condensation de ce brouillard, est également retenu, à la partie supérieure de la deuxième enceinte 40, par un filtre 43 qui offre une ultime surface de condensation aux gouttelettes de milieu liquide éventuellement encore présentes dans le milieu gazeux juste avant que celui-ci ne quitte la deuxième enceinte 40 par le conduit 50.

À sa partie inférieure, la deuxième enceinte 40 est logée et maintenue par vissage dans un bloc support 44 qui entoure le conduit 45, ce conduit reliant, en effet, la base de la deuxième enceinte 40 à la paroi 23a de la chambre de nébulisation 23 de la première enceinte 20, sensiblement à hauteur de l'orifice 32 de la buse de nébulisation 31 et de l'ouverture 33b du tuyau de nébulisation 33.

À sa partie supérieure, la deuxième enceinte 40 comprend un couvercle 46, qui est logé et maintenu dans cette enceinte par vissage et qui est muni d'un orifice 47 au niveau duquel vient se raccorder le conduit 50 servant à évacuer le milieu gazeux hors de ladite enceinte.

Comme visible encore sur la Figure 2, le réservoir 21 est muni de moyens 22 de mesure spectrophotométrique et/ou fluorimétrique.

Pour des mesures spectrophotométriques, ces moyens 22 comprennent un ou plusieurs dispositifs qui comportent chacun, d'une part, une source lumineuse (UV ou visible) permettant d'éclairer le milieu liquide contenu dans le réservoir 21 à une longueur d'onde prédéterminée, et, d'autre part, un capteur permettant de mesurer l'intensité de la lumière transmise par le milieu liquide, lequel capteur est situé dans le même plan horizontal que la source lumineuse mais en vis-à-vis de celle-ci.

La source lumineuse comprend, par exemple, une photodiode qui est située au niveau du réservoir 21 et qui est reliée au système d'alimentation en énergie électrique 80 présent dans le compartiment 15b, ou bien une fibre optique dont l'une des extrémités est située au niveau du réservoir 21 et dont l'autre extrémité est reliée à une photodiode qui, elle, est placée dans le compartiment 15b.

De manière similaire, le capteur peut comprendre un photomultiplicateur qui est situé au niveau du réservoir 21 (auquel cas, le signal transmis par ce photomultiplicateur est un signal électrique), ou bien une fibre optique dont l'une des extrémités est située au niveau du réservoir 21 et dont l'autre extrémité est reliée à un photomultiplicateur qui, lui, est situé sur l'un des circuits électroniques programmés présents dans le compartiment 15b (auquel cas, le signal transmis par cette fibre optique est un signal optique).

Une ou plusieurs lentilles convergentes peuvent également être prévues de manière à ce que toute la lumière transmise par le milieu liquide soit bien captée par le capteur.

Pour des mesures fluorimétriques, les moyens 22 comprennent un ou plusieurs dispositifs analogues à ceux qui viennent décrits à ceci près que, la fluorescence émise par une substance étant similaire dans toutes les directions, le capteur est, de préférence, placé dans le même plan horizontal que la source lumineuse mais transversalement à cette source pour s'affranchir en partie du signal dû au faisceau lumineux émis par la source lumineuse. Par ailleurs, la source lumineuse est dotée d'un filtre optique (passe-haut ou à bande passante) permettant de filtrer la longueur d'onde d'excitation.

La détection d'un explosif présent dans un milieu gazeux tel que l'air ambiant, au moyen de l'appareil 10 qui vient d'être décrit se fait de la manière suivante.

On remplit tout d'abord le réservoir 21 d'un volume prédéterminé d'un milieu liquide contenant au moins une substance capable d'induire, par réaction ou interaction avec cet explosif ou avec un produit résultant de dégradation de cet explosif par un agent chimique, une variation d'un paramètre physico-chimique de ce milieu liquide, et, le cas échéant, un agent chimique capable de dégrader cet explosif.

Puis, on assemble le réservoir à la chambre de nébulisation 23. On actionne la cellule à effet Peltier pour refroidir les première et deuxième enceintes 20 et 40 ainsi que le ou les systèmes de détection 22.

On met en marche la pompe aspirante 60, ce qui a pour effet de mettre en dépression l'intérieur des première et deuxième enceintes 20 et 40 et, partant, de créer une aspiration du milieu gazeux de l'extérieur de la mallette 11 vers la première enceinte 20 (comme illustré par la flèche f₁ sur la Figure 1).

Le milieu gazeux pénètre alors dans la chambre de nébulisation 23 par le conduit 35 en un flux gazeux continu dont on règle le débit au moyen du débitmètre 61.

De par l'action conjuguée de la dépression qui règne dans la première enceinte 20 et de l'augmentation, par la buse de nébulisation 31, de la vitesse à laquelle le flux gazeux est libéré dans la chambre de nébulisation 23, le milieu liquide contenu dans le réservoir 21 est aspiré dans le tuyau de nébulisation 33 par effet Venturi et libéré par l'ouverture 33b que comporte ce tuyau à sa partie supérieure, juste au-dessus de l'orifice 32 de la buse de nébulisation 31.

Il se forme ainsi, dans la chambre de nébulisation 23, un brouillard composé de fines gouttelettes de milieu liquide en suspension dans le milieu gazeux et qui permet, par un contact intime et homogène, entre le milieu liquide et le milieu gazeux, à tous les éléments présents dans le milieu gazeux et ce, quel que soit l'état dans lequel ils se trouvent (gazeux liquide et/ou solide), de se dissoudre dans le milieu liquide et d'être ainsi transférés dudit milieu gazeux dans le milieu liquide.

Le contact intime entre le milieu liquide et le milieu gazeux permet de plus à l'explosif de réagir ou d'interagir avec la substance destinée à permettre sa détection ou bien d'être dégradé par l'agent chimique présent dans le milieu liquide, auquel cas c'est l'un des produits de dégradation qui réagit ou interagit avec ladite substance.

Le brouillard s'élève vers le dôme de condensation 24. Une partie de ces gouttelettes se condense par coalescence au contact de la surface mouillée de la paroi du dôme de condensation et forme des gouttes qui s'écoulent par gravité le long de cette paroi et rejoignent, ainsi, le réservoir 21.

Simultanément, le brouillard, qui ne s'est pas condensé dans la première enceinte 20, s'échappe de cette dernière par le conduit 45, traverse l'élément cylindrique 41 et pénètre dans la deuxième enceinte 40 où les gouttelettes de milieu liquide qui le constituent se condensent au contact de la surface froide des éléments du garnissage 42 et de la paroi 40a de cette deuxième enceinte. Cette condensation se traduit également par la formation de gouttes qui s'écoulent par gravité dans le conduit 45, puis le long de la portion de la paroi 23 qui sépare ce conduit du réservoir 21, et rejoignent ce réservoir.

Ainsi, au fur et à mesure que le milieu liquide contenu dans le réservoir 21 est nébulisé, ce réservoir est alimenté en milieu liquide, à la fois par les gouttes qui se forment dans le dôme de condensation 24 et par celles qui se forment dans la deuxième enceinte 40, ce qui permet de réaliser la nébulisation en continu et de concentrer progressivement, dans le milieu liquide, les éléments initialement présents dans le milieu gazeux ou ceux qui se forment dans le brouillard produit par la nébulisation et ce, aussi longtemps qu'on le souhaite.

Le milieu gazeux quitte, quant à lui, la deuxième enceinte 40 par le conduit 50 (comme illustré par la flèche f₂ sur la Figure 2), après avoir traversé le filtre 43, épuré et asséché.

Pour mettre fin à l'opération d'extraction-concentration, il suffit d'arrêter la pompe aspirante 61, ce qui a pour effet immédiat de stopper l'amenée du milieu gazeux dans la première enceinte 20, l'effet Venturi, l'aspiration du milieu liquide dans le tuyau de nébulisation 33 et la génération du brouillard.

Compte tenu de la présence du ou des moyens de mesure 22, il est possible :
- d'attendre la fin de l'opération d'extraction-concentration pour mesurer le paramètre physico-chimique du milieu liquide dont on sait qu'il va être modifié si l'explosif recherché est présent dans le milieu gazeux ; ou bien
- d'effectuer une mesure de ce paramètre physico-chimique à intervalles de temps, réguliers ou non, pendant que se déroule l'opération d'extraction ; ou bien encore
- d'effectuer une mesure de ce paramètre physico-chimique en continu pendant toute la durée de l'opération d'extraction et de concentration de l'explosif dans le milieu liquide de manière à disposer d'un système d'alerte.

Dans tous les cas, le résultat de la mesure est comparé à un ou plusieurs blancs ayant été préalablement établis en effectuant des opérations d'extraction-nébulisation avec un milieu liquide de composition identique et un milieu gazeux de même nature mais exempt de toute trace d'explosif.

Le procédé de l'invention a été validé au moyen des tests de détection ci-après, qui ont été réalisés en utilisant un appareil tel qu'illustré sur la Figure 2, dans lequel la première enceinte 20 mesure 24 cm de haut sur 9 cm de diamètre et est munie d'un réservoir 21 de 8 cm sur 5 cm de diamètre, ce réservoir est muni de photodiodes pour réaliser des mesures par spectrophotométrie, tandis que la deuxième enceinte 40 est équipée d'un garnissage 42 constitué de billes en acier inoxydable.

### Test 1 : Détection de vapeurs de 2,4-DNT diluées dans l'air ambiant

Un test de détection de vapeurs de 2,4-DNT diluées dans l'air ambiant a été réalisé en utilisant, comme milieu liquide, 25 mL d'une solution d'hydroxyde de tributylammonium à 6% en volume dans de l'acétonitrile.

En présence d'hydroxyde de tributylammonium et d'acétonitrile, le 2,4-DNT se transforme, en effet, en un anion de Janowsky de couleur rouge, dont le pic d'absorption se situe à 517 nm et qui est donc aisément détectable par spectrophotométrie.

Dans le cadre de ce test, une première opération de nébulisation-concentration, d'une durée de trente minutes, a été réalisée en utilisant l'air ambiant comme milieu gazeux, cet air étant exempt de 2,4-DNT, pour obtenir un blanc.

Puis, une deuxième opération de nébulisation-concentration, d'une durée de vingt-cinq minutes, a été réalisée en utilisant comme milieu gazeux, l'air ambiant, toujours exempt de 2,4-DNT, pendant les sept premières minutes, puis en ajoutant à cet air du 2,4-DNT, à hauteur de 30 ppb en volume.

Tous les milieux gazeux ont été utilisés à un débit de 3,2 L/minute.

Pendant toute la durée des opérations de nébulisation-concentration, la température des enceintes 20 et 40 a été maintenue à 5°C et la densité optique du milieu liquide a été mesurée à la longueur d'onde de 500 nm.

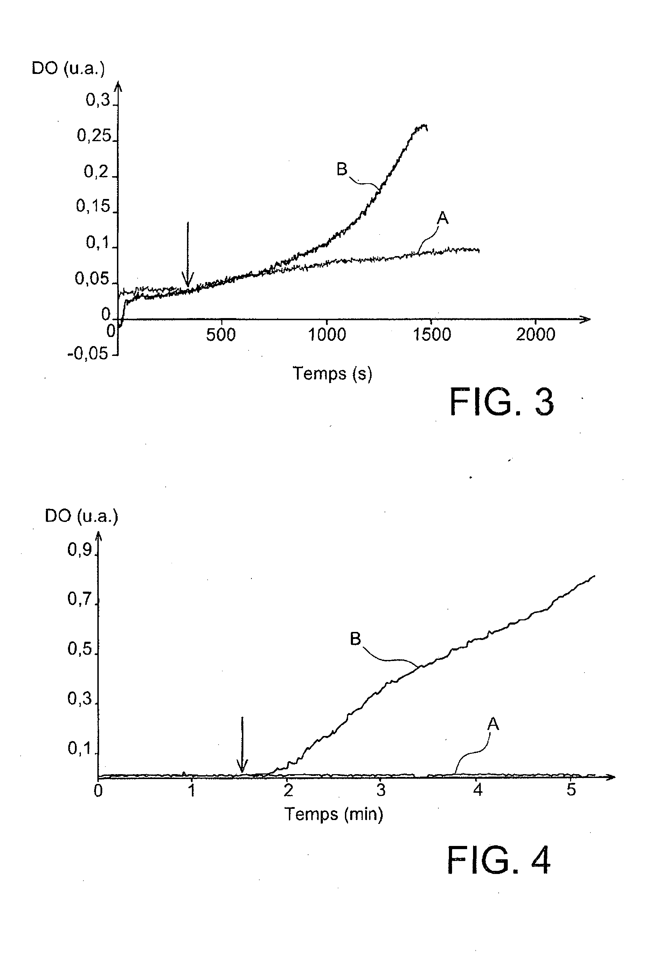

Les résultats de ce test sont illustrés sur la Figure 3 sur laquelle la courbe A correspond à l'évolution de la densité optique obtenue au cours de la première opération de nébulisation-concentration (blanc), tandis que la courbe B correspond à l'évolution de la densité optique obtenue au cours de la deuxième opération de nébulisation-concentration, l'addition du 2,4-DNT dans l'air ambiant étant matérialisée par la flèche noire.

Cette figure montre que, dans le cas du blanc, la densité optique a légèrement augmenté, de manière progressive et régulière, au cours du temps. Cette augmentation de densité optique est liée au fait qu'il s'est produit une légère perte de solvant (acétonitrile) au cours de l'opération de nébulisation-concentration.

Elle montre qu'une augmentation de la densité optique similaire a été observée (et pour les mêmes raisons) pendant les douze premières minutes de la deuxième opération de nébulisation-concentration mais que la densité optique a augmenté de manière drastique au cours des treize minutes restantes de cette opération.

Ce qui signifie que la détection des vapeurs de 2,4-DNT présentes dans l'air ambiant est intervenue cinq minutes après l'addition du 2,4-DNT dans cet air.

Ainsi, non seulement le procédé de l'invention permet de détecter du 2,4-DNT, qui est présent, à l'état gazeux, dans l'air ambiant à l'état de traces (30 ppb), mais il permet de plus de réaliser cette détection dans un laps de temps très court à partir du lancement de l'opération de détection.

### Test 2 : Détection de vapeurs de peroxyde d'hydrogène diluées dans l'air ambiant

Un test de détection de vapeurs de peroxyde d'hydrogène diluées dans l'air ambiant a été réalisé en utilisant, comme milieu liquide, 25 mL d'une solution comprenant de l'oxosulfate de titane(IV), de formule TiOSO₄, de façon à être à 5% massique en titane. On ajoute de l'acide sulfurique, à raison de 3,8 mol/L pour stabiliser l'oxosulfate de titane, de l'eau et de l'acétonitrile dans un rapport volumique eau/acétonitrile de 50/50.

Le peroxyde d'hydrogène forme, en effet, avec l'oxosulfate de titane(IV) un complexe de couleur jaune, dont le pic d'absorption se situe à 410 nm, et qui est donc aisément détectable par spectrophotométrie.

Une première opération de nébulisation-concentration, d'une durée de cinq minutes, a été réalisée en utilisant comme milieu gazeux, l'air ambiant, cet air étant exempt de peroxyde d'hydrogène, pour obtenir un blanc.

Puis, une deuxième opération de nébulisation-concentration, d'une durée de cinq minutes, a été réalisée en utilisant comme milieu gazeux, l'air ambiant, toujours exempt de peroxyde d'hydrogène, pendant les cent premières secondes, puis en ajoutant à cet air du peroxyde d'hydrogène, à hauteur de 75 ppm (parties par million) en volume.

Tous les milieux gazeux ont été utilisés à un débit de 3,2 L/minute.

Pendant toute la durée des opérations de nébulisation-concentration, la température des enceintes 20 et 40 a été maintenue à 5°C et la densité optique du milieu liquide a été mesurée à la longueur d'onde de 410 nm.

Les résultats de ce test sont illustrés sur la Figure 4 sur laquelle la courbe A correspond à l'évolution de la densité optique obtenue au cours des cinq minutes de la première opération de nébulisation-concentration (blanc), tandis que la courbe B correspond à l'évolution de la densité optique obtenue au cours des cinq minutes de la deuxième opération de nébulisation-concentration, l'addition du peroxyde d'hydrogène dans l'air ambiant étant matérialisée par la flèche noire.

Cette figure montre que, dans le cas du blanc, la densité optique est restée sensiblement constante au cours du temps alors que, dans le cas de la deuxième opération de nébulisation-concentration, la densité optique a augmenté de manière brutale dans les trente secondes qui ont suivi l'addition du peroxyde d'hydrogène au milieu ambiant (séparation des courbes A et B).

Ce qui signifie que la détection des vapeurs de peroxyde d'hydrogène présentes dans l'air ambiant est intervenue dans les trente secondes suivant l'addition de cet explosif à cet air.

Ainsi, non seulement le procédé de l'invention permet de détecter du peroxyde d'hydrogène qui est présent à l'état gazeux dans l'air ambiant en quantité très faible (75 ppm) mais il permet de plus de réaliser cette détection quasi instantanément à partir du lancement de l'opération de détection.

### Test 3 : Détection de vapeurs de triperoxyde de triacétone diluées dans l'air ambiant

Un test de détection de vapeurs de triperoxyde de triacétone diluées dans l'air ambiant a été réalisé en suivant un protocole opératoire identique à celui suivi pour le test de détection des vapeurs de peroxyde d'hydrogène décrit ci-avant, à ceci près que les 75 ppm de peroxyde d'hydrogène ont été remplacés par 4 ppm de triperoxyde de triacétone.

Le triperoxyde de triacétone est dégradé en peroxyde d'hydrogène par l'acide sulfurique présent dans le milieu liquide et ce peroxyde d'hydrogène forme avec l'oxosulfate de titane(IV) un complexe de couleur jaune, dont le pic d'absorption se situe à 410 nm, et qui est donc aisément détectable par spectrophotométrie.

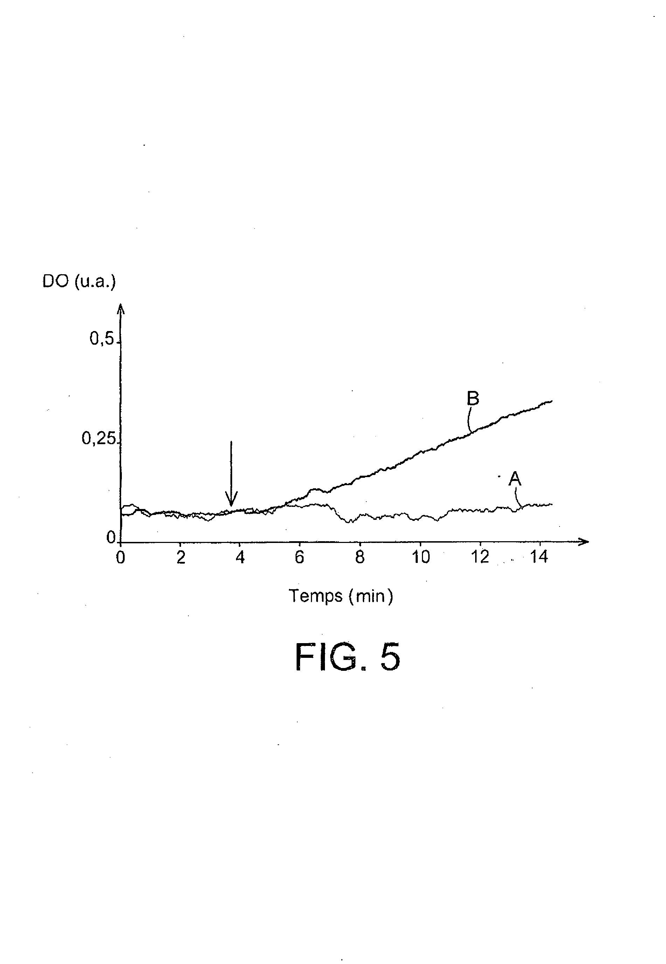

Les résultats de ce test sont illustrés sur la Figure 5 sur laquelle la courbe A correspond à l'évolution de la densité optique obtenue au cours des quatorze premières minutes de la première opération de nébulisation-concentration (blanc), tandis que la courbe B correspond à l'évolution de la densité optique obtenue au cours des quatorze premières minutes de la deuxième opération de nébulisation-concentration, l'addition du triperoxyde de triacétone dans l'air ambiant étant matérialisée par la flèche noire.

Cette figure montre que, dans le cas du blanc, la densité optique est restée sensiblement constante au cours du temps alors que, dans le cas de la deuxième opération de nébulisation-concentration, la densité optique a augmenté de manière brutale une minute et demi après l'addition du triperoxyde de triacétone au milieu ambiant (séparation des courbes A et B).

Ce qui signifie que la détection des vapeurs de triperoxyde de triacétone présentes dans l'air ambiant est intervenue une minute et demie après que cet explosif a été ajouté à cet air.

Ainsi, non seulement le procédé de l'invention permet de détecter du triperoxyde de triacétone qui est présent à l'état gazeux dans l'air ambiant en quantité extrêmement faible (4 ppb) mais il permet de plus de réaliser cette détection quasi instantanément à partir du lancement de l'opération de détection.

### RÉFÉRENCES CITÉES

[1] Brevet US numéro 5,011,517
[2] Cofer III et al., Envir. Sci. Technol. 1985, 19(6), 557-560
[3] Demande internationale PCT publiée sous le numéro WO 2005/025721

## Revendications

1. Procédé pour détecter *in situ* la présence d'au moins un explosif dans un milieu gazeux, lequel procédé comprend une opération d'extraction et de concentration de cet explosif dans un milieu liquide, cette opération comprenant la nébulisation du milieu liquide au moyen du milieu gazeux et la condensation des gouttelettes de milieu liquide formant le brouillard produit par cette nébulisation, et est **caractérisé en ce que** :
- on utilise, comme milieu liquide, un milieu qui contient au moins une substance capable de réagir avec cet explosif ou avec un produit résultant de la dégradation de cet explosif par un agent chimique, et dont la réaction ou l'interaction avec ledit explosif ou ledit produit de dégradation entraîne une variation d'au moins un paramètre physico-chimique du milieu liquide ;
- on mesure ledit paramètre physico-chimique pendant l'opération d'extraction et de concentration de l'explosif ou à la fin de cette opération ; et
- on corrèle le résultat de la mesure dudit paramètre physico-chimique avec la présence ou l'absence de l'explosif dans le milieu gazeux.

2. Procédé selon la revendication 1, dans lequel l'explosif est choisi parmi les explosifs intrinsèques, leurs précurseurs et les marqueurs d'explosifs.

3. Procédé selon la revendication 1 ou la revendication 2, dans lequel l'explosif est choisi parmi les composés nitroaromatiques, les nitramines, les esters nitriques et les peroxydes organiques.

4. Procédé selon l'une quelconque des revendications précédentes, dans lequel le paramètre physico-chimique du milieu liquide est une propriété optique.

5. Procédé selon la revendication 4, dans lequel la propriété optique est la densité optique ou la fluorescence.

6. Procédé selon l'une quelconque des revendications précédentes, dans lequel la substance contenue dans le milieu liquide est choisie parmi les bases fortes et les halogénures de tributylammonium.

7. Procédé selon l'une quelconque des revendications 1 à 5, dans lequel la substance contenue dans le milieu liquide est choisie parmi les composés de titane (IV) .

8. Procédé selon l'une quelconque des revendications 1 à 5, dans lequel la substance contenue dans le milieu liquide est l'oxosulfate de titane.

9. Procédé selon l'une quelconque des revendications 1 à 5, dans lequel la substance contenue dans le milieu liquide est une phtalocyanine, une coumarine ou le dansylamide.

10. Procédé selon la revendication 9, dans lequel la phtalocyanine répond à la formule (I) ci-après : 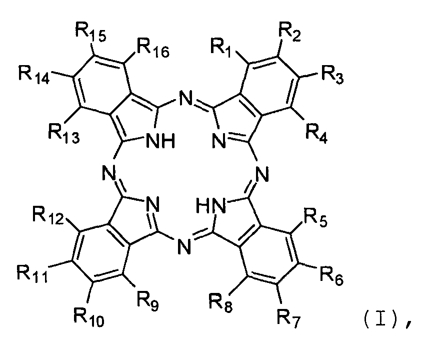 dans laquelle :
- R₁, R₄, R₅, R₈, R₉, R₁₂, R₁₃ et R₁₆, identiques ou différents, représentent un atome d'hydrogène ou un groupe alkyle comprenant de 1 à 4 atomes de carbone et, mieux encore, un groupe méthyle ou éthyle, tandis que R₂, R₃, R₆, R₇, R₁₀, R₁₁, R₁₄ et R₁₅ représentent un groupe alcoxy ; ou bien
- R₁, R₂, R₄, R₅, R₆, R₈, R₉, R₁₀, R₁₂, R₁₃, R₁₄ et R₁₆ représentent un atome d'hydrogène, tandis que R₃, R₇, R₁₁ et R₁₄ représentent un groupe aryloxy ou un groupe silylé ;
cette phtalocyanine pouvant être métallée ou non métallée.

11. Procédé selon l'une quelconque des revendications précédentes, dans lequel le milieu liquide contient de plus au moins un agent chimique capable de dégrader l'explosif et/ou au moins un composé propre à favoriser la solubilisation dans le milieu liquide de l'explosif ou du produit résultant de sa dégradation par l'agent chimique.

12. Procédé selon la revendication 11 dépendante de la revendication 7 ou de la revendication 8, dans lequel l'agent chimique est un acide fort, de préférence de l'acide sulfurique.

13. Procédé selon l'une quelconque des revendications précédentes, dans lequel la mesure du paramètre physico-chimique du milieu liquide est réalisée par spectrophotométrie ou par fluorimétrie.

14. Procédé selon l'une quelconque des revendications précédentes, dans lequel la mesure du paramètre physico-chimique du milieu liquide est effectuée en continu pendant toute la durée de l'opération d'extraction et de concentration de l'explosif dans le milieu liquide.

15. Procédé selon l'une quelconque des revendications précédentes, dans lequel l'opération d'extraction et de concentration de l'explosif dans le milieu liquide comprend une première et une deuxième condensation des gouttelettes de milieu liquide formant le brouillard produit par la nébulisation, la première condensation étant réalisée par coalescence des gouttelettes sur une surface mouillée et la deuxième condensation étant réalisée par contact desdites gouttelettes avec une surface froide.

16. Procédé selon la revendication 15, qui est mis en oeuvre dans un appareil (10) qui comprend :
- une première enceinte (20) de nébulisation et de condensation, qui présente une partie supérieure (24) et une partie inférieure (21) destinée à contenir le milieu liquide, et qui est munie d'un conduit (35) d'amenée du milieu gazeux, de moyens (31, 33) pour nébuliser le milieu liquide, et d'un premier conduit d'évacuation (45) du milieu gazeux ;
- des moyens (60) de mise en dépression ou en surpression de l'intérieur de la première enceinte pour permettre au milieu gazeux de pénétrer dans cette première enceinte, d'y circuler et d'en être évacué, en un flux continu ;
- une deuxième enceinte (40) de condensation, cette deuxième enceinte étant reliée au conduit (45) d'évacuation du milieu gazeux de la première enceinte, et étant munie d'un deuxième conduit (50) d'évacuation du milieu gazeux ; et
- des moyens (52) pour refroidir cette deuxième enceinte ; et
- des moyens (22) pour mesurer le paramètre physico-chimique du milieu liquide dont la variation est induite par la réaction ou l'interaction entre la substance et l'explosif ou le produit résultant de sa dégradation par l'agent chimique.

17. Procédé selon la revendication 16, dans lequel les moyens pour mesurer le paramètre physico-chimique du milieu liquide sont des moyens de mesure spectrophotométrique et/ou fluorimétrique.

## Patentansprüche

1. Verfahren zum In-situ-Nachweis wenigstens eines Explosivstoffs in einem gasförmigen Medium, wobei das Verfahren einen Arbeitsschritt der Extraktion und Konzentrierens dieses Explosivstoffs in einem flüssigen Medium umfasst und dieser Arbeitsschritt die Vernebelung des flüssigen Mediums mittels eines gasförmigen Mediums und die Kondensation der durch diese Vernebelung erzeugten, den Nebel bildenden Tröpfchen aus dem flüssigen Medium umfasst und **dadurch gekennzeichnet ist, dass**:
- als flüssiges Medium ein Medium verwendet wird, das wenigstens eine Substanz enthält, die mit diesem Explosivstoff oder mit einem aus der Zersetzung dieses Explosivstoffs mit einem chemischen Agens entstehenden Produkt zu reagieren vermag und dessen Reaktion oder Wechselwirkung mit dem besagten Explosivstoff oder dem besagten Zersetzungsprodukt eine Änderung wenigstens eines physikalisch-chemischen Parameters des flüssigen Mediums herbeiführt;
- der besagte physikalisch-chemische Parameter während des Arbeitsschrittes der Extraktion und des Konzentrierens des Explosivstoffs oder am Ende dieses Arbeitsschrittes gemessen wird; und
- das Ergebnis der Messung dieses physikalisch-chemischen Parameters mit der Anwesenheit oder Abwesenheit des Explosivstoffs in dem gasförmigen Medium korreliert wird.

2. Verfahren gemäß Anspruch 1, wobei der Explosivstoff aus den eigentlichen Explosivstoffen, ihren Vorläufern und Markierungsmitteln für Explosivstoffe ausgewählt ist.

3. Verfahren gemäß Anspruch 1 oder Anspruch 2, wobei der Explosivstoff aus nitroaromatischen Verbindungen, Nitraminen, Salpetersäureestern und organischen Peroxiden ausgewählt ist.

4. Verfahren gemäß einem der vorangehenden Ansprüche, wobei der physikalisch-chemische Parameter des flüssigen Mediums eine optische Eigenschaft ist.

5. Verfahren gemäß Anspruch 4, wobei die optische Eigenschaft die optische Dichte oder die Fluoreszenz ist.

6. Verfahren gemäß einem der vorangehenden Ansprüche, wobei die in dem flüssigen Medium enthaltene Substanz aus starken Basen und Tributylammoniumhalogeniden ausgewählt ist.

7. Verfahren gemäß einem der Ansprüche 1 bis 5, wobei die in dem flüssigen Medium enthaltene Substanz aus Titan(IV)-Verbindungen ausgewählt ist.

8. Verfahren gemäß einem der Ansprüche 1 bis 5, wobei die in dem flüssigen Medium enthaltene Substanz Titanoxosulfat ist.

9. Verfahren gemäß einem der Ansprüche 1 bis 5, wobei die in dem flüssigen Medium enthaltene Substanz ein Phthalocyanin, ein Coumarin oder Dansylamid ist.

10. Verfahren gemäß Anspruch 9, wobei das Phthalocyanin der nachstehenden Formel (I) entspricht: 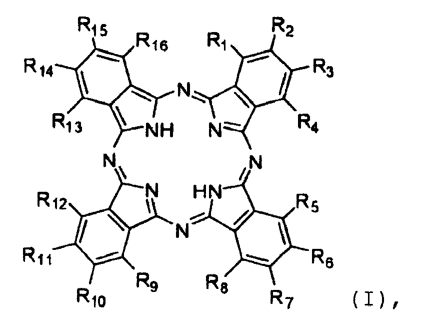 worin:
- R₁, R₄, R₅, R₈, R₉, R₁₂, R₁₃ und R₁₆, die gleich oder verschieden sind, ein Wasserstoffatom oder eine 1 bis 4 Kohlenstoffatome umfassende Alkylgruppe, noch besser eine Methyl- oder Ethylgruppe bedeuten, während R₂, R₃, R₆, R₇, R₁₀, R₁₁ und R₁₅ eine Alkoxygruppe bedeuten; oder
- R₁, R₂, R₄, R₅, R₆, R₈, R₉, R₁₀, R₁₂, R₁₃, R₁₄ und R₁₆ ein Wasserstoffatom bedeuten, während R₃, R₇, R₁₁ und R₁₄ eine Aryloxygruppe oder eine Silylgruppe bedeuten;
wobei dieses Phthalocyanin metalliert oder nicht metalliert sein kann.

11. Verfahren gemäß einem der vorangehenden Ansprüche, wobei das flüssige Medium außerdem wenigstens ein chemisches Agens, das den Explosivstoff zu zersetzen vermag, und/oder eine geeignete Verbindung enthält, um das Lösen des Explosivstoffs oder des sich aus seiner Zersetzung durch das chemische Agens ergebenden Produkts in dem flüssigen Medium zu fördern.

12. Verfahren gemäß dem von Anspruch 7 oder dem Anspruch 8 abhängigen Anspruch 11, wobei das chemische Agens eine starke Säure, vorzugsweise Schwefelsäure ist.

13. Verfahren gemäß einem der vorangehenden Ansprüche, wobei die Messung des physikalisch-chemischen Parameters des flüssigen Mediums durch Spektrophotometrie oder durch Fluorimetrie durchgeführt wird.

14. Verfahren gemäß einem der vorangehenden Ansprüche, wobei die Messung des physikalisch-chemischen Parameters des flüssigen Mediums kontinuierlich während der Gesamtdauer des Arbeitsschrittes der Extraktion und des Konzentrierens des Explosivstoffes in dem flüssigen Medium durchgeführt wird.

15. Verfahren gemäß einem der vorangehenden Ansprüche, wobei der Arbeitsschritt der Extraktion und des Konzentrierens des Explosivstoffs in dem flüssigen Medium ein erstes und zweites Kondensieren der durch diese Vernebelung erzeugten, den Nebel bildenden Tröpfchen aus dem flüssigen Medium umfasst, die erste Kondensierung durch Koaleszenz der Tröpfchen auf einer feuchten Oberfläche bewerkstelligt wird und die zweite Kondensation durch Kontakt der besagten Tröpfchen mit einer kalten Oberfläche bewerkstelligt wird.

16. Verfahren gemäß Anspruch 15, das in einer Apparatur (10) durchgeführt wird, die Folgendes umfasst:
- einen ersten Raum (20) zur Vernebelung und zum Kondensieren, der einen oberen Teil (24) und einen unteren Teil (21) aufweist, der dazu bestimmt ist, das flüssige Medium aufzunehmen und der mit einer Leitung (35) für die Zufuhr des gasförmigen Mediums, Mitteln (31, 33) zum Vernebeln des flüssigen Mediums und einer ersten Leitung (45) zur Abführung des gasförmigen Mediums versehen ist;
- Mittel (60) zum Beaufschlagen des Inneren des ersten Raumes mit Unterdruck oder Überdruck, um dem gasförmigen Medium das Eindringen in diesen ersten Raum zu gestatten, dort in konstantem Strom zu zirkulieren und daraus abgeführt zu werden;
- einen zweiten Raum (40) zum Kondensieren, wobei dieser zweite Raum mit der Leitung (45) zur Abführung des gasförmigen Mediums aus dem ersten Raum verbunden ist und mit einer zweiten Leitung (50) zur Abführung des gasförmigen Mediums versehen ist; und
- Mittel (52) zum Kühlen dieser zweiten Leitung; und
- Mittel (22) zum Messen des physikalisch-chemischen Parameters des flüssigen Mediums, dessen Änderung durch die Reaktion oder Wechselwirkung zwischen der Substanz und dem Explosivstoff oder dem sich aus seiner Zersetzung durch das chemische Agens ergebenden Produkt bewirkt wird.

17. Verfahren gemäß Anspruch 16, wobei die Mittel zum Messen des physikalisch-chemischen Parameters des flüssigen Mediums Mittel zur spektrophotometrischen und/oder fluorimetrischen Messung sind.

## Claims

1. Method for the *in situ* detection of the presence of at least one explosive in a gaseous medium, which method comprises an operation for extracting and concentrating this explosive in a liquid medium, this operation comprising nebulisation of the liquid medium by means of the gaseous medium and condensation the droplets of liquid medium forming the mist produced by this nebulisation, and is **characterised in that**:
- as the liquid medium, a medium which contains at least one substance capable of reacting with this explosive or with a product resulting from the degradation of this explosive by a chemical agent, and whose the reaction or interaction with said explosive or said degradation product induces a variation of at least one physicochemical parameter of the liquid medium is used;
- said physicochemical parameter is measured during the operation for extracting and concentrating the explosive or at the end of this operation; and
- the result of the measurement of said physicochemical parameter is correlated with the presence or absence of the explosive in the gaseous medium.

2. Method according to claim 1, wherein the explosive is chosen from intrinsic explosives, precursors thereof and explosive markers.

3. Method according to claim 1 or claim 2, wherein the explosive is chosen from nitroaromatic compounds, nitramines, nitric esters and organic peroxides.

4. Method according to any of the preceding claims, wherein the physicochemical parameter of the liquid medium is an optical property.

5. Method according to claim 4, wherein the optical property is the optical density or fluorescence.

6. Method according to any of the preceding claims, wherein the substance contained in the liquid medium is chosen from strong bases and tributylammonium halides.

7. Method according to any of claims 1 to 5, wherein the substance contained in the liquid medium is chosen from titanium(IV) compounds.

8. Method according to any of claims 1 to 5, wherein the substance contained in the liquid medium is titanium oxosulphate.

9. Method according to any of claims 1 to 5, wherein the substance contained in the liquid medium is a phthalocyanine, a coumarin or dansylamide.

10. Method according to claim 9, wherein the phthalocyanine complies with formula (I) hereinafter: 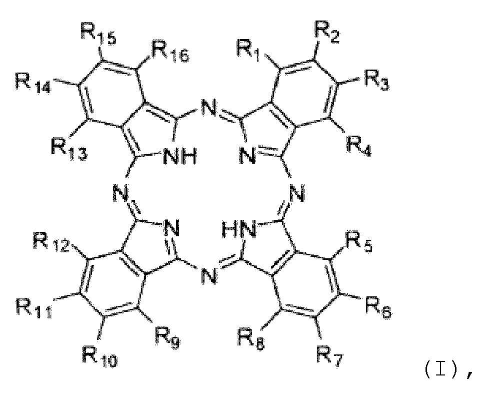 wherein:
- R₁, R₄, R₅, R₈, R₉, R₁₂, R₁₃ and R₁₆, identical or different, represent a hydrogen atom or an alkyl group comprising 1 to 4 carbon atoms and, more preferably, a methyl or ethyl group, whereas R₂, R₃, R₆, R₇, R₁₀, R₁₁, R₁₄ and R₁₅ represent an alkoxy group; or
- R₁, R₂, R₄, R₅, R₆, R₈, R₉, R₁₀, R₁₂, R₁₃, R₁₄ and R₁₆ represent a hydrogen atom, whereas R₃, R₇, R₁₁ and R₁₄ represent an aryloxy group or a silylated group;
this phthalocyanine optionally being metallised or non-metallised.

11. Method according to any of the preceding claims, wherein the liquid medium further contains at least one chemical agent capable of degrading the explosive and/or at least one compound suitable for promoting the solubilisation in the liquid medium of the explosive or the product resulting from the degradation thereof by the chemical agent.

12. Method according to claim 11 dependent on claim 7 or claim 8, wherein the chemical agent is a strong acid, preferably sulphuric acid.

13. Method according to any of the preceding claims, wherein the measurement of the physicochemical parameter of the liquid medium is made by means of spectrophotometry or by means of fluorimetry.

14. Method according to any of the preceding claims, wherein the measurement of the physicochemical parameter of the liquid medium is performed continuously throughout the operation for extracting and concentrating the explosive in the liquid medium.

15. Method according to any of the preceding claims, wherein the operation for extracting and concentrating the explosive in the liquid medium comprises a first and a second condensation of the droplets of liquid medium forming the mist produced by the nebulisation, the first condensation being performed by coalescence of the droplets on a wetted surface and the second condensation being performed by contact of said droplets with a cold surface.

16. Method according to claim 15, used in an apparatus (10) comprising:
- a first enclosure (20) for nebulisation and condensation, which has an upper part (24) and a lower part (21) intended to contain the liquid medium, and which is provided with a conduit (35) for delivering the gaseous medium, means (31, 33) for nebulising the liquid medium, and a first conduit (45) for discharging the gaseous medium;
- means (60) for depressurising or pressurising the interior of the first enclosure to allow the gaseous medium to enter this first enclosure, circulate therein and be discharged therefrom, in a continuous flow;
- a second enclosure (40) for condensation, this second enclosure being connected to the conduit (45) for discharging the gaseous medium from the first enclosure, and being provided with a second conduit (50) for discharging the gaseous medium; and
- means (52) for cooling this second enclosure; and
- means (22) for measuring the physicochemical parameter of the liquid medium whose the variation is induced by the reaction or interaction between the substance and the explosive or the product resulting from the degradation thereof by the chemical agent.

17. Method according to claim 16, wherein the means for measuring the physicochemical parameter of the liquid medium are spectrophotometric and/or fluorimetric measurement means.
